# EUROPEAN PATENT SPECIFICATION

(11) **EP 1 729 685 B1**
(45) Date of publication and mention of the grant of the patent: **15.07.2015**
(21) Application number: 05730849.6
(22) Date of filing: 28.03.2005
(51) Int. Cl.: A61F 2/07, A61F 2/848, A61F 2/89, A61F 2/95, A61F 2/954, A61F 2/24, A61F 2/06

(54) **ENDOLUMINAL GRAFT WITH A PROSTHETIC VALVE**
ENDOLUMINALE PROTHESE MIT KÜNSTLICHER KLAPPE
GREFFE ENDOLUMINALE AVEC VALVE PROTHETIQUE

(30) Priority: 31.03.2004 US 558169 P
(43) Date of publication of application: 13.12.2006
(73) Proprietor: Cook Medical Technologies LLC, Bloomington, IN 47404 (US)
(72) Inventor: DEL PAINE, Stepanie, West Lafayette, IN 47906 (US)
(74) Representative: Jehan, Robert
(86) International application number: PCT/US2005/010354
(87) International publication number: WO 2005/096993

(56) References cited:
- WO-A-01/66043
- US-A- 5 895 419
- US-A1- 2002 107 565
- US-A1- 2003 120 333
- US-A1- 2003 120 333
- US-A1- 2003 199 967
- US-A1- 2004 034 408

## Description

### BACKGROUND OF THE INVENTION

### 1. Technical Field.

This invention relates to a medical device and, in particular, a prosthesis for implantation within the human or animal body for the repair of a damaged endoluminal valve, such as an aortic valve, and a method for implanting the same.

### 2. Related Art.

Throughout this specification, when discussing the aorta or other blood vessels, the terms distal and distally with respect to a prosthesis are intended to refer to the end of the prosthesis furthest away in the direction of blood flow from the heart. Similarly, the terms proximal and proximally are intended to mean the end of the prosthesis which, when implanted, would be nearest to the heart.

The aortic valve functions as a one-way valve between the heart and the rest of the body. Blood is pumped from the left ventricle of the heart, through the aortic valve, and into the aorta, which in turn supplies blood to the body. Between heart contractions the valve closes, preventing blood from flowing backwards into the heart. The function of the aortic valve is twofold. First, it provides a route for which blood can leave the heart. Second, it prevents blood that has already left the heart from leaking backwards into the heart.

Damage to the aortic valve can occur from a congenital defect, the natural aging process, and from infection or scarring. Certain types of damage may cause the aortic valve to "leak", resulting in "aortic insufficiency" or "aortic regurgitation." Aortic regurgitation causes an extra workload for the heart, and can ultimately result in weakening of the heart muscle and eventual heart failure. After the aortic valve becomes sufficiently damaged, the valve may need to be replaced to prevent heart failure and death.

An open heart operation to replace a defective aortic valve can take between two and three hours to perform. During the procedure, the damaged valve is removed and replaced with either a biological tissue valve or a "mechanical" valve. Although tissue and mechanical valves function similarly, there are distinct advantages and disadvantages of each. One advantage of mechanical valves, which are generally made from ceramic materials, is that they may last forever. A disadvantage of mechanical valves is that they can require anticoagulation with blood thinners for the remainder of a patient's life. One advantage of tissue valves, which are made from cow or pig hearts, is that they may not require formal anticoagulation. A disadvantage of tissue valves, however, is that they generally wear out after 12-15 years, at which time another operation can be required to replace the worn out valve.

During an aortic valve replacement procedure, it may be necessary or desirable to reinforce a portion of the aorta adjacent to the valve with a graft. It would be desirable to provide a single device that would act as both a supplemental or replacement aortic valve and also as a reinforcing stent. It would be further desirable to provide a minimally intrusive method for implanting such a device.

The deployment of intraluminal prostheses into the lumen of a patient from a remote location by the use of a deployment device or introducer has been disclosed in a number of earlier patents and patent applications. United States Patent No. 4,562,596 entitled "Aortic Graft, Device and Method for Performing an Intraluminal Abdominal Aortic Aneurysm Repair" proposes the retention of a self-expanding graft within a sleeve until it is to be deployed, at which time the sleeve is withdrawn and the graft is allowed to expand.

United States Patent No. 4,665,918 entitled "Prosthesis System and Method" proposes a system and method for the deployment of a prosthesis in a blood vessel. The prosthesis is positioned between a delivery catheter and an outer sheath and expands outwardly upon removal of the sheath.

United States Patent No. 4,950,227 entitled "Stent Delivery System" proposes the delivery of a stent by mounting the stent to the outside of an inflatable catheter and retaining the ends of an unexpanded stent by fitting a sleeve over either end of the stent. Expansion of the stent is caused by inflation of the catheter between the sleeves so that the ends of the stent are withdrawn from the respective sleeves and the stent released and expanded into position.

United States Patent No. 5,387,235 entitled "Expandable Transluminal Graft Prosthesis for Repair of Aneurysm" discloses apparatus and methods of retaining grafts onto deployment devices.

United States Patent No. 5,720,776 entitled "Barb and Expandable Transluminal Graft Prosthesis for Repair of Aneurysm" discloses improved barbs with various forms of mechanical attachment to a stent.

United States Patent No. 6,206,931 entitled "Graft Prosthesis Materials" discloses graft prosthesis materials and a method for implanting, transplanting replacing and repairing a part of a patient and particularly the manufacture and use of a purified, collagen based matrix structure removed from a submucosa tissue source.

PCT Patent Publication Number No. WO99/29262 entitled "Endoluminal Aortic Stents" discloses a fenestrated prosthesis for placement where there are intersecting arteries.

PCT Patent Publication Number No. WO03/034948 entitled "Prostheses for Curved Lumens" discloses prostheses with arrangements for bending the prosthesis for placement into curved lumens.

United States Patent Application Publication No. 2003/0233140 entitled "Trigger Wire System" discloses release wire systems for the release of stent grafts retained on introducer devices.

United States Patent Application Publication No. 2004/0098079 entitled "Thoracic Aortic Stent Graft Deployment Device" discloses introducer devices adapted for deployment of stent grafts particularly in the thoracic arch.

United States Patent Application Publication No. 2004/0054396 entitled "Stent-Graft Fastening" discloses arrangements for fastening stents onto grafts particularly for exposed stents.

PCT Patent Publication Number No. WO03/053287 entitled "Stent Graft with Improved Graft Adhesion" discloses arrangements on stent grafts for enhancing the adhesion of such stent grafts into walls of vessels in which they are deployed.

PCT Patent Publication Number No. WO98/53761 entitled "A Prosthesis and a Method and Means of Deploying a Prosthesis", discloses various embodiments of an introducer for positioning an expandable endovascular prosthesis in a lumen of a patient.

### SUMMARY

An endoluminal prosthesis is provided for providing replacement for a damaged endoluminal valve restricting fluid flow in a lumen. The intraluminal prosthesis comprises a tubular graft having a flexible body including an inner surface that defines an inner volume, a self-expanding stent mounted to the proximal end of the flexible body, the self-expanding stent extending beyond the flexible body proximal end, and a valve prosthesis at the proximal end of the flexible body, the valve prosthesis including at least one leaflet having an edge coupled to the proximal end of the flexible body, the or each leaflet having a free edge responsive to pressure differentials and movable between an open position permitting a fluid flow through the inner volume and a closed position restricting fluid flow through the inner volume.

The one or more stents can be self-expanding stents. One stent can be mounted to a proximal end of the flexible body of the tubular graft, and can extend beyond the proximal end. Additionally, one stent can be mounted to a distal end of the flexible body of the tubular graft, and can extend beyond the distal end. The one or more stents can include attachment barbs.

The tubular graft can be a body portion of a thoracic aortic prosthetic device, a renal prosthetic device, a superior mesenteric prosthetic device, a celiac prosthetic device, or the like. The prosthetic valve can comprise one or more leaflets. The one or more leaflets can include a derived collagen material, such as an extracellular matrix. The extracellular matrix can be small intestinal submucosa, stomach submucosa, pericardium, liver basement membrane, urinary bladder submucosa, tissue mucosa, dura mater, or the like. The one or more leaflets can also include a synthetic material, such as a polyester or polytetrafluoroethylene. The prosthetic valve can be coupled to the tubular graft with suture.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.
FIG 1 is a partial cut-away view of a heart and an aorta.
FIG 2 is a perspective view of an endoluminal prosthesis.
FIG 2A is a perspective view of another endoluminal prosthesis.
FIG 3 is a bottom plan view of the endoluminal prosthesis of FIG 2.
FIG 4 is a cutaway side view of the endoluminal prosthesis of FIG 2 showing a valve prosthesis located therein.
FIG 5 is an inverted sectional view of the valve prosthesis of FIG 4 in a "closed" condition.
FIG 6 is a section view similar to FIG 5 showing the valve prosthesis in an "open" condition.
FIG 7 is an exploded perspective view of an introducer of a prosthesis partially deployed; the introducer not forming part of the invention.
FIG 8 is a sectional view of a portion of the introducer of FIG 7 around the proximal end of the prosthesis.
FIG 9 is a sectional view of a portion of the introducer of FIG 7 around the distal end of the prosthesis.
FIG 10 is a sectional view of a portion of the introducer of FIG 7 around the haemostatic seal.
FIG 11 is a sectional view of a portion of the introducer of FIG 7 around the trigger wire release mechanisms.
FIG 12 is a sectional view of a portion of the introducer of FIG 7 around the pin vise clamp and the medical reagent introduction tube.
FIG 13 is an exploded sectional view of the introducer of FIG 7 fully loaded and ready for introduction into a patient.
FIG 14 is an exploded view partially in section of the introducer of FIG 13 in the next stage of deployment of the prosthesis.
FIG 15 is an exploded view partially in section of the introducer of FIG 13 with the release of the proximal end stage of deployment.
FIG 16 is an exploded view partially in section of the introducer of FIG 13 with the release of the distal end stage of deployment.
FIG 17 is a view similar to FIG 16 showing the advancement of the distal attachment mechanism to the proximal attachment mechanism.
FIG 18 is a view similar to FIG 16 showing the withdrawal of the introducer.
FIG 19 is a partial cut-away view of the heart and the aorta of FIG 1 with the endoluminal prosthesis of FIG 2 situated in the aorta.
FIG 20 is a bottom plan view of a second endoluminal prosthesis.
FIG 21 is a cutaway side view of the prosthesis of FIG 20 showing the valve prosthesis located therein in a "closed" condition.
FIG 22 is a cutaway side view of the prosthesis of FIG 20 in an "open" condition.
FIG 23 is a bottom plan view of a third endoluminal prosthesis.
FIG 24 is a cutaway perspective view of the prosthesis of FIG 23 showing the valve prosthesis located therein in an "open" condition.
FIG 25 shows the prosthesis of FIG 23 in a "closed" condition.

### DETAILED DESCRIPTION OF THE PREFERRED EMBODIMENTS

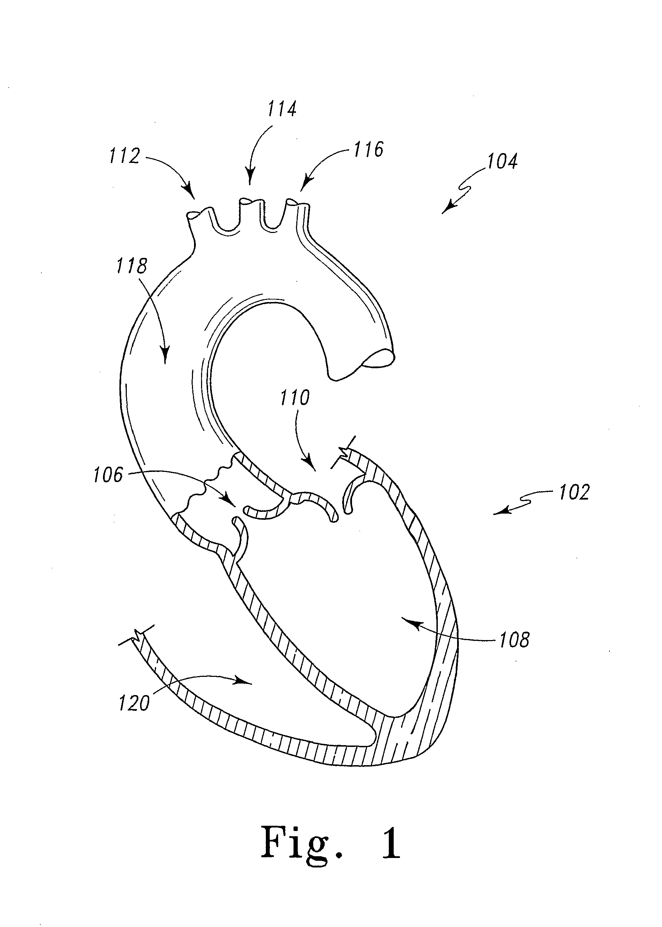

FIG 1 is a partial cut-away view of a heart 102 and an aorta 104. The heart 102 can have an aortic valve 106 that does not seal properly. This defect of the aortic valve 106 allows blood to flow from the aorta 104 back into the left ventricle 108, leading to a disorder known as aortic regurgitation. A bicuspid mitral valve 110 generally prevents blood from flowing further backwards into the left atrium, not shown. Also shown in FIG 1 are a brachioephalic trunk 112, a left common carotid artery 114, a left subclavian artery 116, and a right ventricle 120. A portion of the aorta 104 referred to herein as an ascending aorta 118 is shown located between the aortic valve 106 and brachioephalic trunk 112.

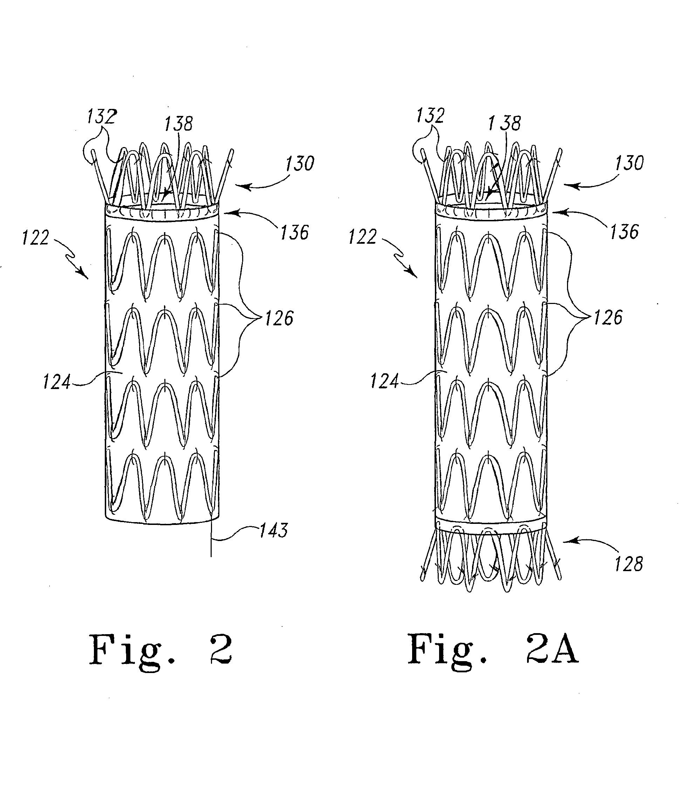

FIG 2 is a perspective view of an endoluminal prosthesis 122. The term "prosthesis" means any replacement for a body part or function of that body part. It can also mean a device that enhances or adds functionality to a physiological system. The terms "endoluminal" and "intraluminal" describe objects that are found or can be placed inside a lumen in the human or animal body. A lumen can be an existing lumen or a lumen created by surgical intervention. This includes lumens such as blood vessels, parts of the gastrointestinal tract, ducts such as bile ducts, parts of the respiratory system, etc. "Endoluminal prosthesis" thus describes a prosthesis that can be placed inside one of these lumens.

The prosthesis 122 comprises a tubular graft material 124, with self-expanding stents 126 attached thereto. The term "graft" means the generally annular or tubular member which acts as an artificial vessel. A graft by itself or with the addition of other elements can be an endoluminal prosthesis. The term "stent" means any device or structure that adds rigidity, expansion force or support to a prosthesis.

The tubular graft material 124 is preferably non-porous so that it does not leak or sweat under physiologic forces. The graft material is preferably made of woven DACRON® polyester (VASCUTEK® Ltd., Renfrewshire, Scotland, UK). The tubular graft can be made of any other at least substantially biocompatible material including such fabrics as other polyester fabrics, polytetrafluoroethylene (PTFE), expanded PTFE, and other synthetic materials. Naturally occurring biomaterials, such as collagen, are also highly desirable, particularly a derived collagen material known as ex-tracellular matrix (ECM), such as small intestinal submucosa (SIS).

Other examples of ECMs are pericardium, stomach submucosa, Liver basement membrane, urinary bladder submucosa, tissue mucosa, and dura mater. SIS is particularly useful, and can be made in the fashion described in U.S. Patent No. 4,902,508 to Badylak et al.; U.S. Patent No. 5,733,337 to Carr; 17 Nature Biotechnology 1083 (Nov. 1999); and WIPO Publication WO 98/22158 of May 28, 1998, to Cook et al., which is the published application of PCT/US97/14855. All of these patents and publications are incorporated herein by reference.

Irrespective of the origin of the graft material (synthetic versus naturally occurring), the graft material can be made thicker by making multi-laminate constructs, for example SIS constructs as described in U.S. Patent Nos. 5,968,096; 5,955,110; 5,885,619; and 5,711,969. All of these patents are incorporated herein by reference. In addition to xenogenic biomaterials, such as SIS, autologous tissue can be harvested as well, for use in forming the graft material. Additionally elastin or elastin-like polypeptides (ELPs) and the like offer potential as a material to fabricate the graft material.

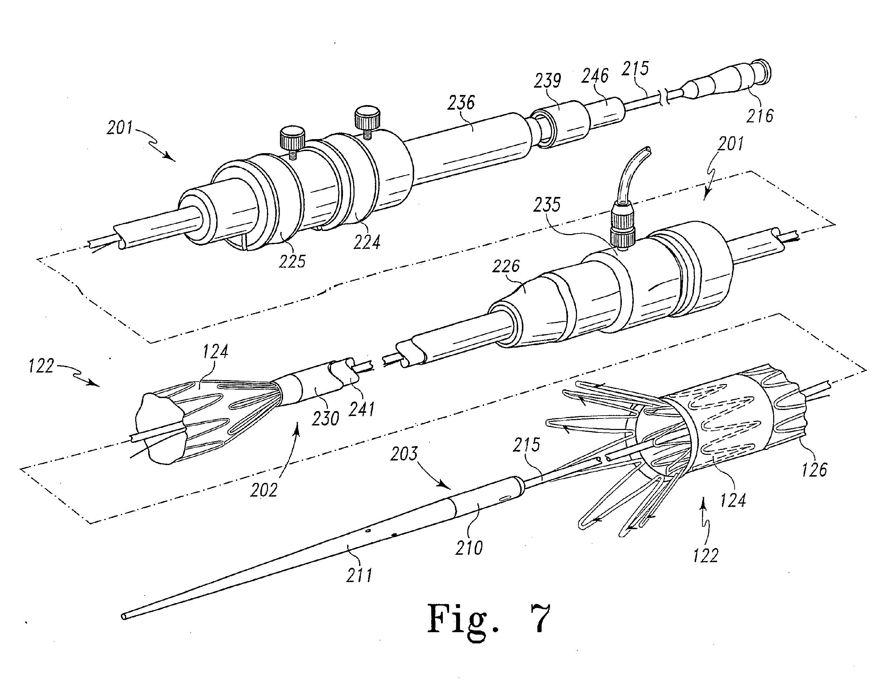

The self-expanding stents 126 cause the prosthesis 122 to expand following its disengagement from an introducer 201, shown in FIG 7. The prosthesis 122 can include a proximal self-expanding zigzag stent 130 that extends from a proximal end of the prosthesis 122. As shown in FIG 2A, a second embodiment of the prosthesis 122 can also include a distal self-expanding zigzag stent 128 that extends from a distal end of the prosthesis 122. The proximal and distal self-expanding zigzag stent 128 and 130 can each include barbs 132 that can aid in anchoring the prosthesis 122 to the lumen.

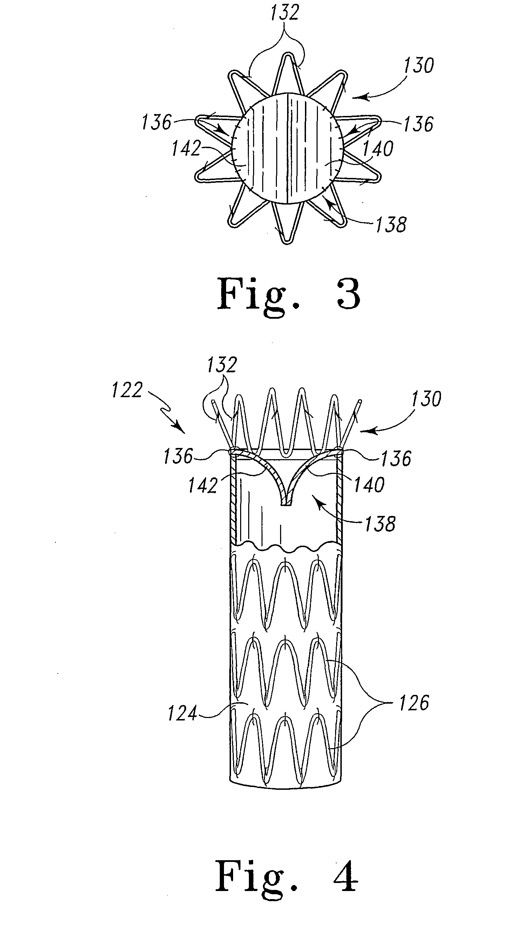

The prosthesis 122 further comprises fasteners 136 that secure a valve prosthesis 138, which is shown in FIG 3. The fasteners 136 can be any material suitable for securing a valve prosthesis 138 to the intraluminal prosthesis 122, such as metal rings, hinges, or the like. The fasteners 136 are preferable one or more sutures.

The valve prosthesis 138 can include a first leaflet 140 and a second leaflet 142. The first and second leaflets 140 and 142 can be fabricated from any at least substantially biocompatible material including, such materials as other polyester fabrics, polytetrafluoroethylene (PTFE), expanded PTFE, and other synthetic materials known to those of skill in the art. Preferably, the first and second leaflets 140 and 142 are fabricated from naturally occurring biomaterials, such as SIS or another ECM, as discussed above. The SIS can be made in one of the fashions described above.

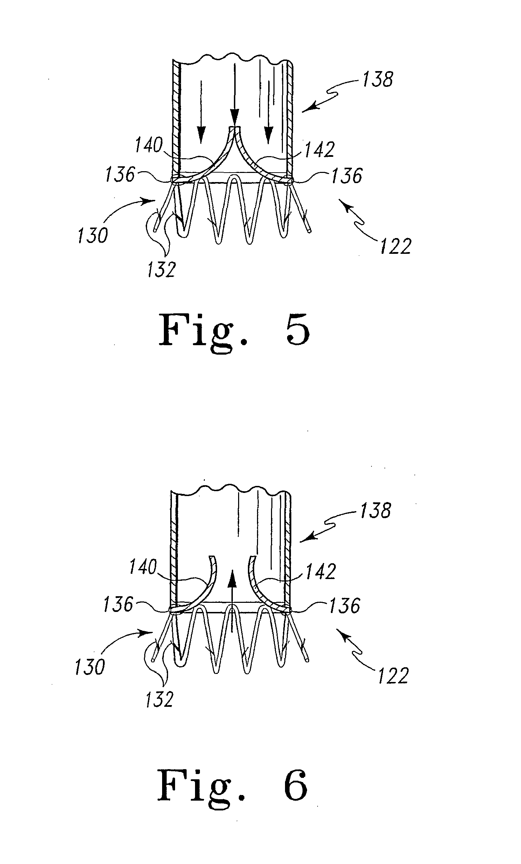

The first and second leaflets 140 and 142 are arranged in the intraluminal prosthesis 122 shown in FIG 4 such that the leaflets mimic a naturally occurring bicuspid valve. As shown in FIG 5, the valve prosthesis 138 is normally "closed". As shown in FIG 6, however, the valve prosthesis 138 "opens" to allow blood flow when the pressure on the proximal side of the valve prosthesis 138 is greater than pressure on the distal side of the valve prosthesis 138.

FIG 7 shows an endovascular deployment system, also known as an introducer, for deploying the intraluminal prosthesis 122 in a lumen of a patient during a medical procedure. The introducer includes an external manipulation section 201, a distal positioning mechanism attachment region 202 and a proximal positioning mechanism attachment region 203. During the medical procedure to deploy the prosthesis 122, the distal and proximal attachment regions 202 and 203 will travel through the lumen to a desired deployment site. The external manipulation section 201, which is acted upon by a user to manipulate the introducer, remains outside of the patient throughout the procedure.

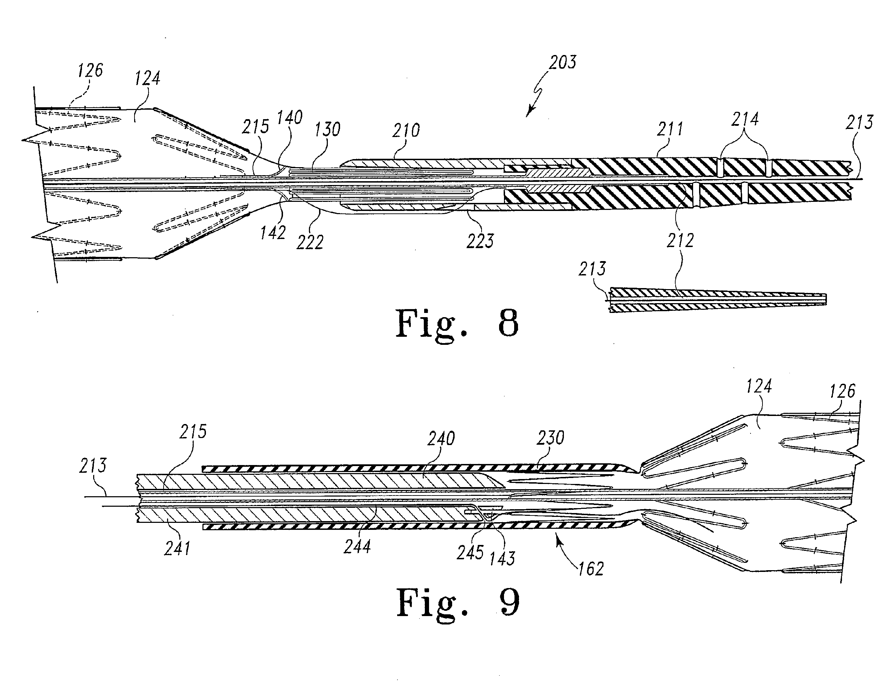

FIG 8 shows the proximal attachment region 203 in greater detail. The proximal attachment region 203 includes a cylindrical sleeve 210. The cylindrical sleeve 210 has a long tapered flexible extension 211 extending from its proximal end. The flexible extension 211 has an internal longitudinal aperture 212. The longitudinal aperture 212 facilitates advancement of the tapered flexible extension 211 along an insertion wire 213. The aperture 212 also provides a channel for the introduction of medical reagents, which will flow through openings 214. For example, it may be desirable to supply a contrast agent to allow angiography to be performed during placement and deployment phases of the medical procedure.

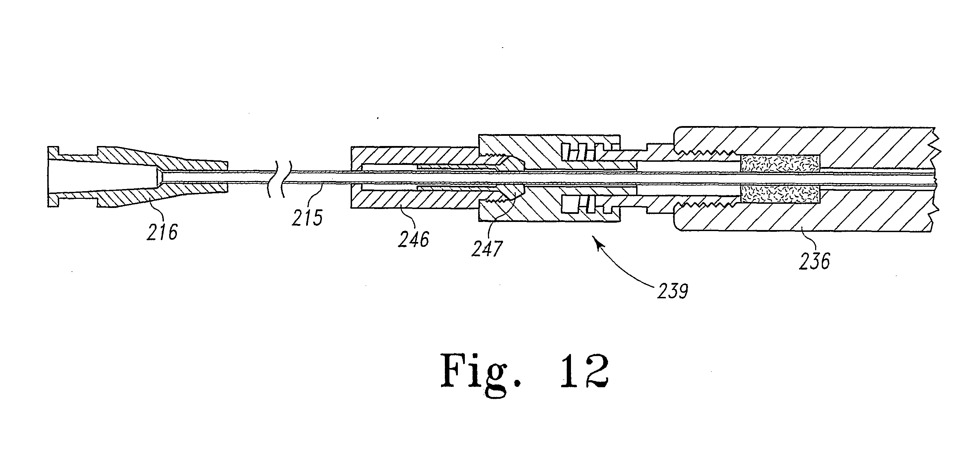

A thin walled tube 215 is fastened to the extension 211. The thin-walled tube 215 is sufficiently flexible so that the introducer can be advanced along a relatively tortuous vessel, such as a femoral artery. The thin-walled tube 215 also allows manipulation longitudinally and rotationally of the proximal attachment region 203. The thin-walled tube 215 extends through the introducer to the manipulation section 201, terminating at a connection means 216, as shown in FIG 12.

Regarding the introduction of reagents, FIG 12 also shows that the connection means 216 is adapted to accept a syringe to facilitate the introduction of reagents into the tube 215. The tube 215 is in fluid communication with the aperture 212 of the flexible extension 211. Therefore, reagents introduced into connection means 216 flow through the aperture 212 and emanate from the openings 214.

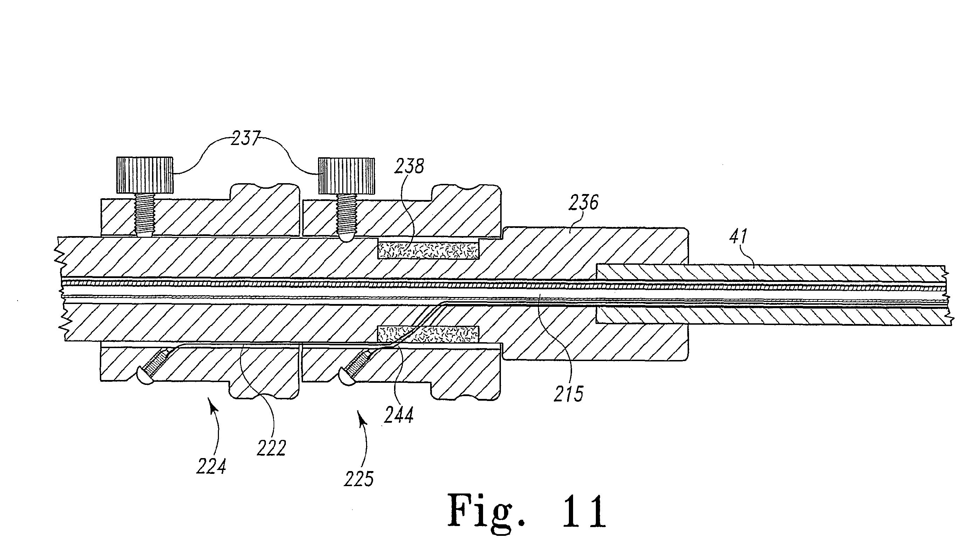

As shown in FIG 9, a tube 241 is coaxial with and radially outside the thin-walled tube 215. The tube 241 is "thick-walled", that is to say the thickness of its wall is several times that of the thin-walled tube 215. A sheath 230 is coaxial with and radially outside the thick-walled tube 241. The thick-walled tube 241 and the sheath 230 extend distally to the manipulation region 1, as shown in FIGs 7 and 11.

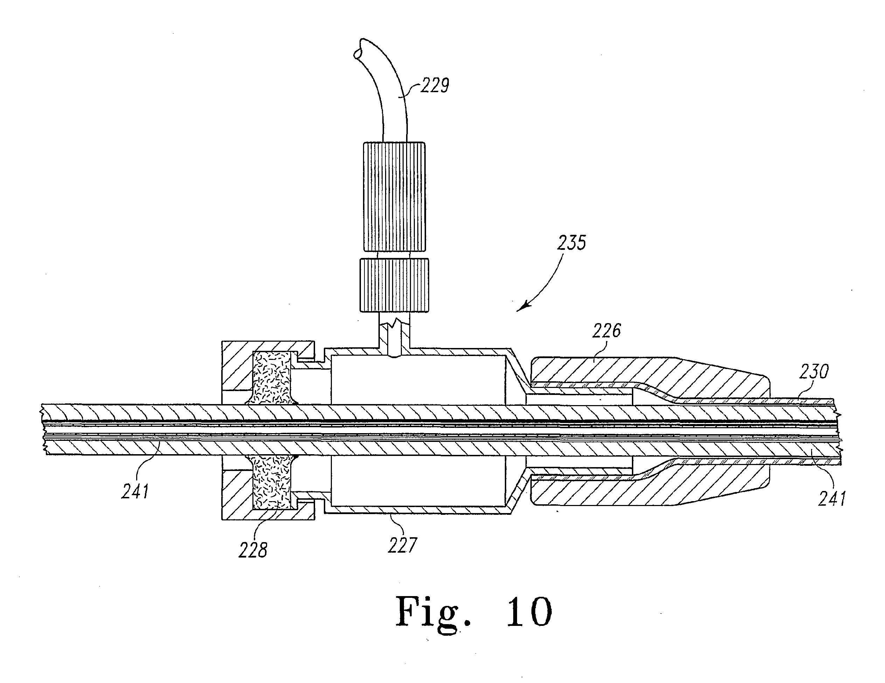

FIGs 8 and 9 illustrate distal and proximal retention and release mechanisms of the introducer. During the placement phase of the medical procedure, the prosthesis 122 is retained in a compressed condition by the sheath 230. The sheath 230 extends distally to a gripping and haemostatic sealing means 235 of the external manipulation section 201, shown in FIG 10.

During assembly of the introducer, the sheath 230 is advanced over the cylindrical sleeve 210 of the proximal attachment region 203 while the prosthesis 122 is held in a compressed state by an external force. A distal attachment section 240 is formed in the thick-walled tube 241 to retain the distal end of the prosthesis 122. Alternatively, the distal attachment section 240 can be a separate piece coupled to the thick-walled tube 241.

The self-expanding stent 130 is released by retracting the sheath 230, removing the trigger wire 222, and then sliding the proximal attachment region 203, including the retention device 210, proximally away from the stent 130. Once the retention device 210 has cleared the self-expanding stent 130, the stent 130 will expand. The trigger wire 222 and the proximal wire release mechanism 224 form a control member to selectively release the retention device 210 from the prosthesis 122 by holding the self-expanding stent 130 in the retention device 210 until the prosthesis 122 is positioned at a desired site in the lumen.

The distal end 162 of the prosthesis 122 is retained by the distal attachment section 240 of the thick-walled tube 241. The distal end 162 of the prosthesis 122 has a loop 143 through which a distal trigger wire 244 extends. The distal trigger wire 244 extends through an aperture 245 in the distal attachment section 240 into the annular region between the thin-walled tube 215 and the thick-walled tube 241.

As shown in FIG 11, the distal trigger wire 244 extends through the annular space between the thick-walled tube 241 and the thin-walled tube 215 to the manipulation region 201. The distal trigger wire 244 exits the annular space at a distal wire release mechanism 225. The distal trigger wire 244 and the distal wire release mechanism 225 form a control member to selectively disengage the distal retention section 240 from the prosthesis 122 when it is positioned at a desired site in the lumen.

FIG 10 shows the haemostatic sealing means 235 of the external manipulation section 201 in greater detail. The haemostatic sealing means 235 includes a haemostatic seal 227 and a side tube 229. The haemostatic seal 227 includes a clamping collar 226 that clamps the sheath 230 to the haemostatic seal 227. The haemostatic seal 227 also includes a silicone seal ring 228. The silicone seal ring 228 forms a haemostatic seal around the thick-walled tube 241. The side tube 229 facilitates the introduction of medical reagents between the thick-walled tube 241 and the sheath 230.

FIG 11 shows a proximal portion of the external manipulation section 201. The release wire actuation section has a body 236 that is mounted onto the thick-walled tube 241. The thin-walled tube 215 passes through the body 236. The distal wire release mechanism 225 is mounted for slidable movement on the body 236. Similarly, the proximal wire release mechanism 222 is mounted for slidable movement on the body 236. A pair of clamping screws 237 prevents inadvertent early release of the prosthesis 122.

The positioning of the proximal and distal wire release mechanisms 224 and 225 is such that the proximal wire release mechanism 224 must be moved before the distal wire release mechanism 225 can be moved. Therefore, the distal end 162 of the prosthesis 122 cannot be released until the self-expanding zigzag stent 130 has been released and anchored to the lumen. A haemostatic seal 238 is provided so the release wires 222 and 244 can extend out through the body 236 to the release mechanisms 224 and 225 without unnecessary blood loss during the medical procedure.

FIG 12 shows a distal portion of the external manipulation section 201. A pin vise 239 is mounted onto the distal end of the body 236. The pin vise 239 has a screw cap 246. When screwed in, the vise jaws 247 clamp against and engage the thin-walled tube 215. When the vise jaws 247 are engaged, the thin-walled tube 215 can only move with the body 236, and hence the thin-walled tube 215 can only move with the thick-walled tube 241. With the screw cap 246 tightened, the entire assembly, except for the external sleeve 230, can be moved as one.

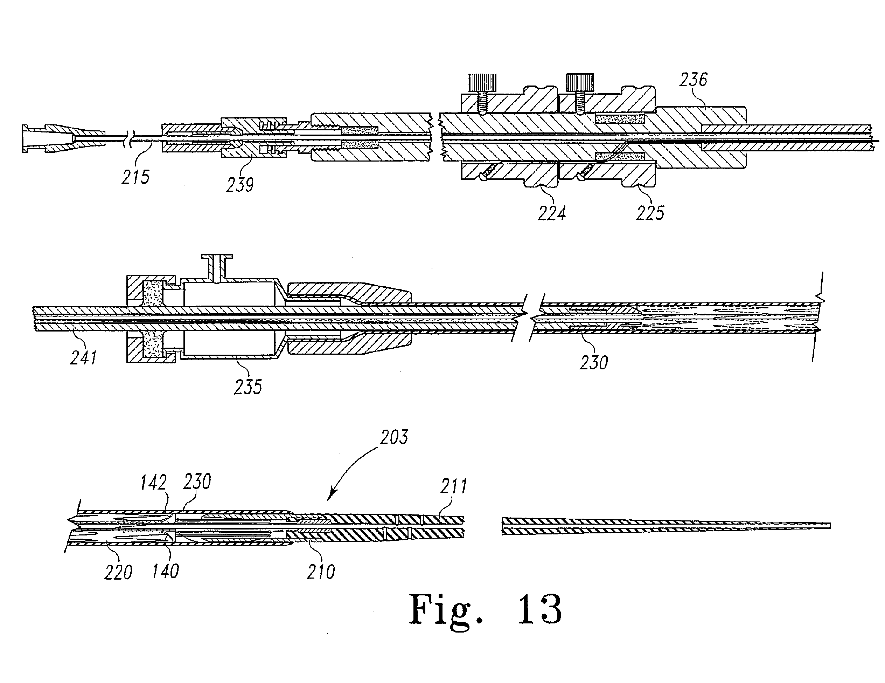

The prosthesis 122 can be deployed in any method known in the art, preferably the method described in WO98/53761, in which the device is inserted by an introducer via a surgical cut-down into a femoral artery, and then advanced into the desired position over a stiff wire guide using endoluminal interventional techniques. For example, FIGS 13 through 18 show various stages of the deployment of the prosthesis 122 during an illustrative medical procedure. A guide wire 213, seen in FIGs 8 and 9, is introduced into the femoral artery and advanced until its tip is beyond the region into which the prosthesis 122 is to be deployed.

In FIG 13, the introducer assembly is shown fully assembled ready for introduction into a patient. The prosthesis 122 is retained at each of its ends by the proximal and distal retaining assemblies respectively, and compressed by the external sleeve 230. For example, the introducer assembly can be inserted through a femoral artery over the guide wire 213 and positioned in the aorta by well known radiographic techniques not discussed here.

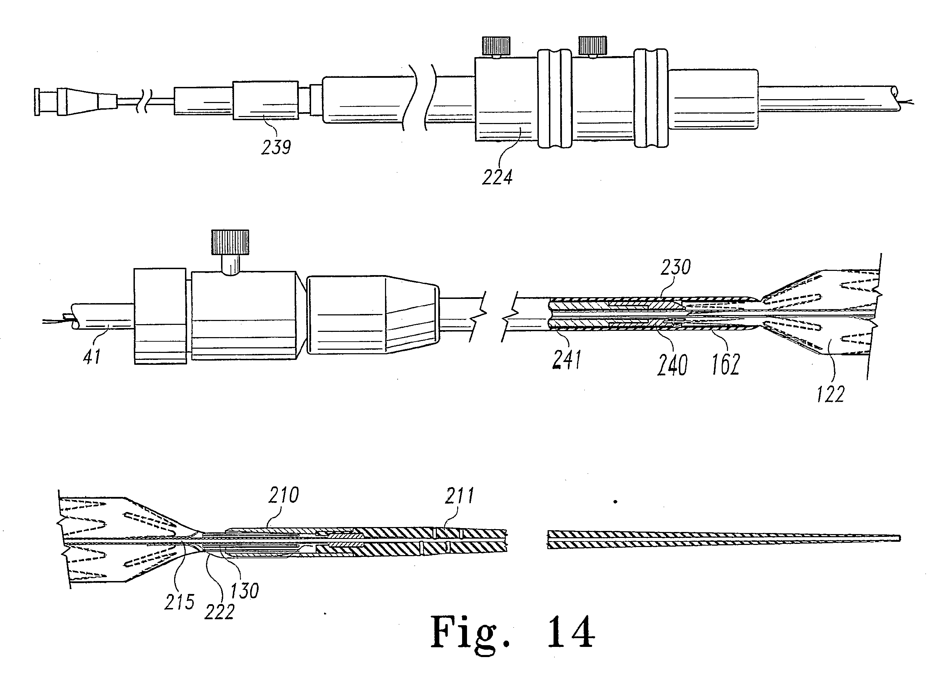

Once the introducer assembly is in a desired position for deployment of the prosthesis 122, as shown in FIG 14, the external sheath 230 can be withdrawn to just proximal of the distal attachment section 240. This action releases the middle portion of the prosthesis 122 so that it can expand radially. The proximal self-expanding stent 130, however, is still retained within the retention device 210. Also, the distal end 162 of the prosthesis 122 is still retained within the external sheath 230.

By release of the pin vise 239 to allow small movements of the thin-walled tubing 215 with respect to the thick-walled tubing 241, the prosthesis 122 can be lengthened or shortened or rotated or compressed for accurate placement in the desired location within the aorta. X-ray opaque markers, not shown, can be placed along the prosthesis 122 to assist with placement of the prosthesis.

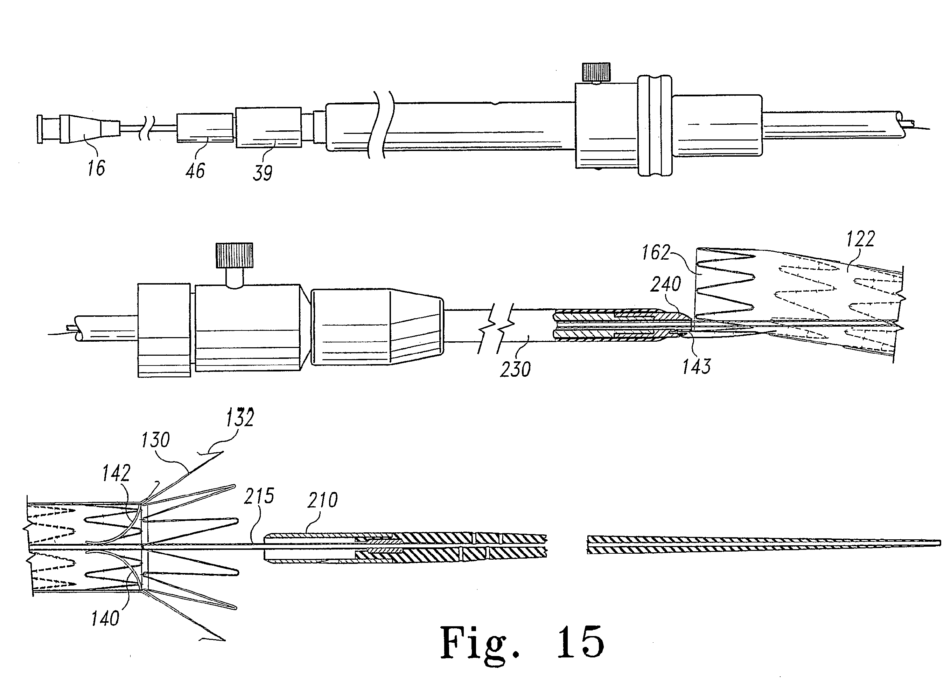

In FIG 15, the proximal trigger wire 222 has been removed, allowing the retention device 210 to be separated from the self-expanding zigzag stent 130, as explained above..At this stage, the proximal trigger wire release mechanism 224 and the proximal trigger wire 222 can be removed completely.

Also, the screw cap 246 of the pin vise 239 has been loosened so that the thin-walled tubing 215 can been pushed in a proximal direction to move the proximal attachment means 210 in a proximal direction. When the proximal attachment means 210 no longer surrounds the self-expanding stent 130 at the proximal end of the prosthesis 122, the self-expanding stent 130 expands. When the self-expanding stent 130 expands, the hooks or barbs 132 on the self-expanding stent 130 grip into the walls of the lumen to hold the proximal end of the prosthesis 122 in place.

At this point, the distal end 162 of the prosthesis 122 is still retained by the distal attachment means 240, with the loop 143 retained therein. The external sheath 230 is then withdrawn to distal of the distal attachment section 240 to allow the distal end 162 of the prosthesis 122 to expand. At this point, the distal end 162 of the prosthesis 122 can still be moved. Consequently, the prosthesis 122 can still be rotated or lengthened or shortened or otherwise moved for accurate positioning.

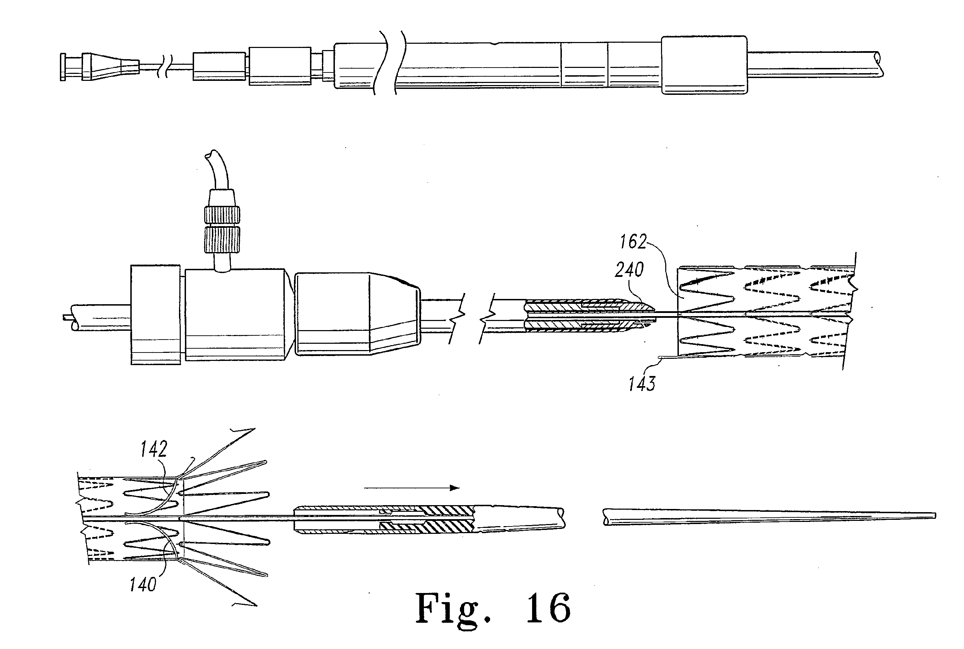

In FIG 16, the distal end 162 of the prosthesis 122 has been released by removal of the distal trigger wire 244. At this stage, the distal trigger wire release mechanism 225 and the distal trigger wire 244 can be removed completely. This removal can be accomplished by passing the distal wire release mechanism 225 over the pin vise 239 and the connection means 216. The loop 143 of the terminal distal self-expanding zigzag stent 126 is hence released, and the prosthesis 122 is now free to expand to the walls of the vessel. At this point, the introducer is ready to be removed.

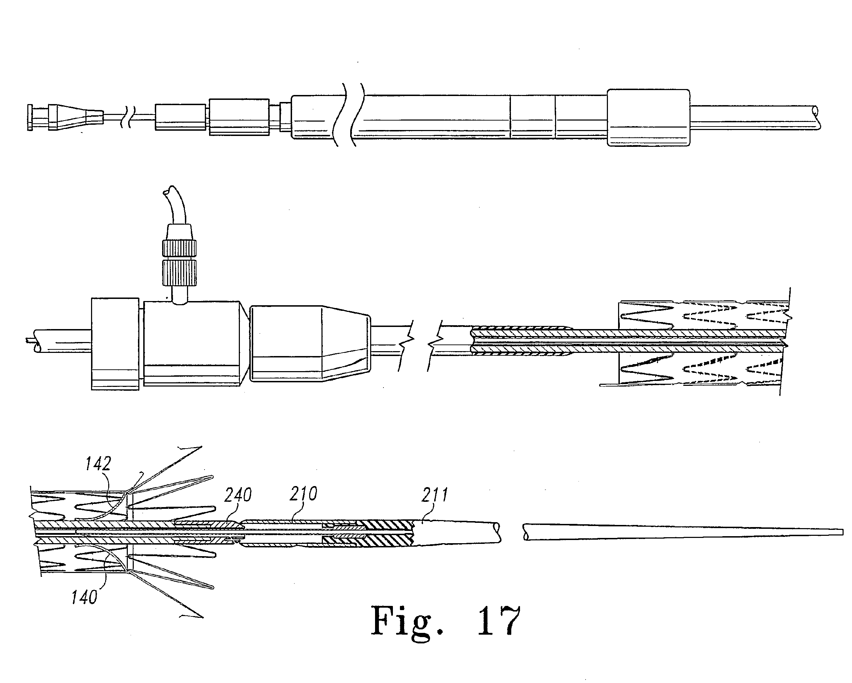

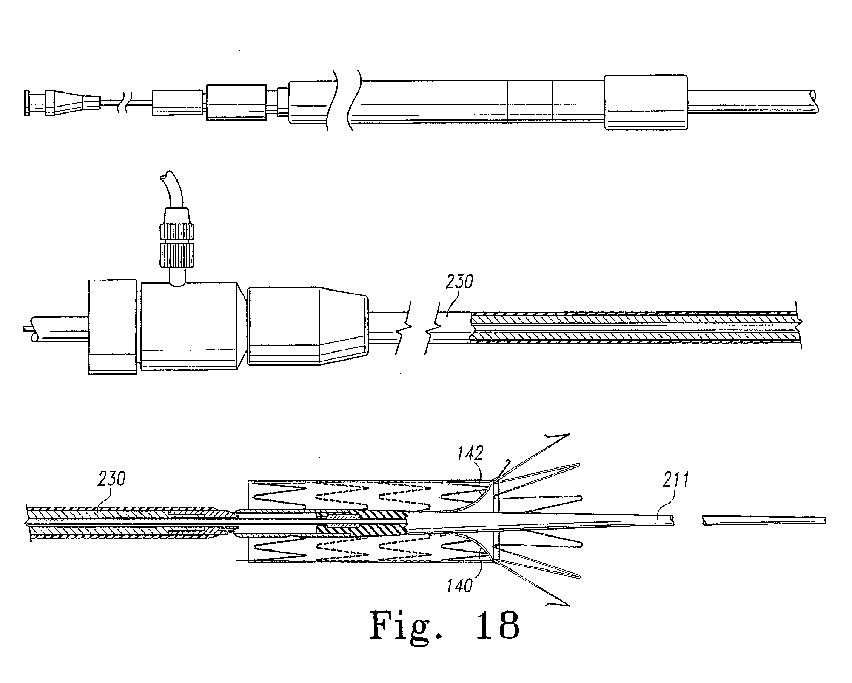

In FIG 17, the first stage of removal is shown. First, the distal attachment section 240 is advanced until it is received in the rear of the proximal attachment device 210. Next, the proximal attachment device 210, the tapered flexible extension 211, and the distal attachment device 240 are removed together, as shown in FIG 18.

In FIG 18, the sheath 230 has been advanced to uncover the joint between the proximal attachment device 210 and the distal attachment section 240. The sheath 230 can be removed with the proximal attachment device 210, the tapered flexible extension 211 and the distal attachment device 240. Alternatively, these items could be removed separately, followed by removal of the external sleeve 230.

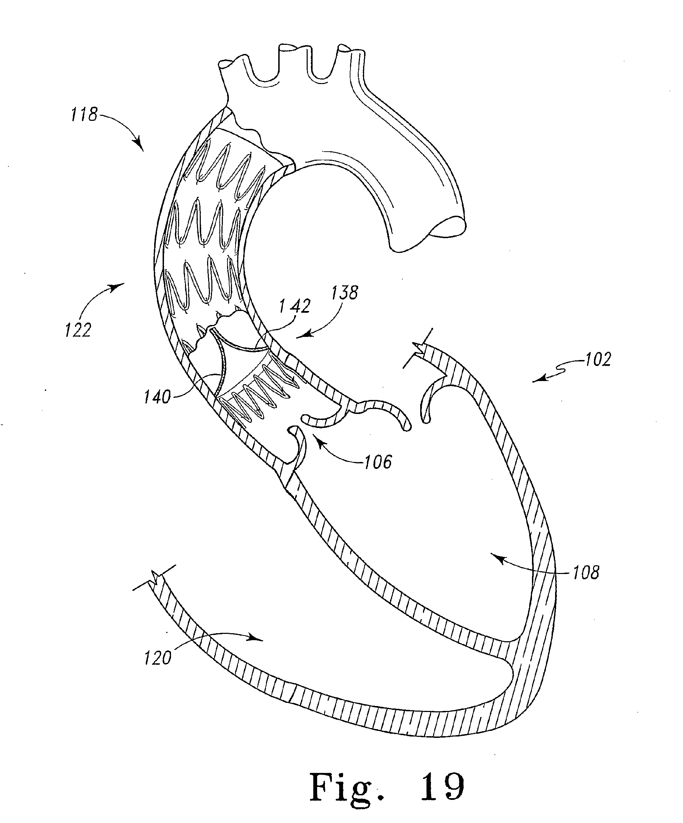

In FIG 19 is a cutaway view of the ascending aorta 118 showing the prosthesis 122 placed in a position to supplement the aortic valve 106. Supplementing the aortic valve 106 is only one exemplary use of the claimed invention, and the use of this example is not intended to limit the claimed invention in any way. In FIG 19 also shows the prosthesis 122 cut away to reveal the first and second leaflets 140 and 142 of the valve prosthesis 138, which is shown closed.

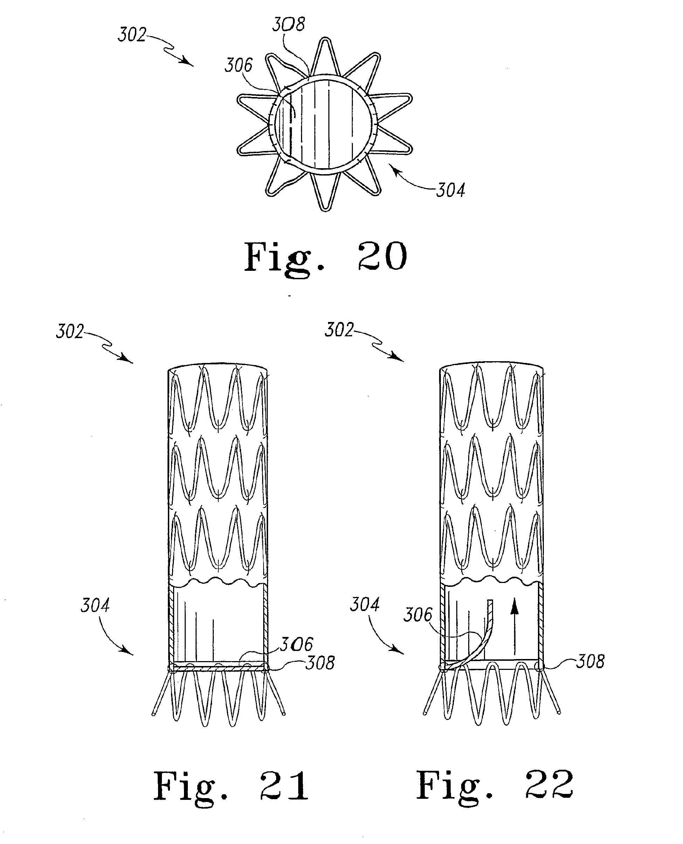

The second intraluminal prosthesis 302 shown in FIG 20 includes a valve prosthesis 304, but is otherwise similar to the intraluminal prosthesis 122. The valve prosthesis 304 can include a flap member 306 and a stop member 308. The flap member 306 and the stop member 308 can be fabricated from any at least substantially biocompatible material including, such materials as other polyester fabrics, polytetrafluoroethylene (PTFE), expanded PTFE, and other synthetic materials known to those of skill in the art. Preferably, the flap member 306 and the stop member 308 are fabricated from naturally occurring biomaterials, such as SIS or another ECM, as discussed above.

FIG 21 is a cutaway side view of the intraluminal prosthesis 302 showing the valve prosthesis 304 located therein in a "closed" position. The flap member 306 and the stop member 308 are arranged in the intraluminal prosthesis 302 such that the flap member 306 can move to allow fluid to flow in one direction. As shown in FIG 21, the valve prosthesis 304 is normally closed. As shown in FIG 22, however, the valve prosthesis 304 "opens" to allow fluid flow under pressure.

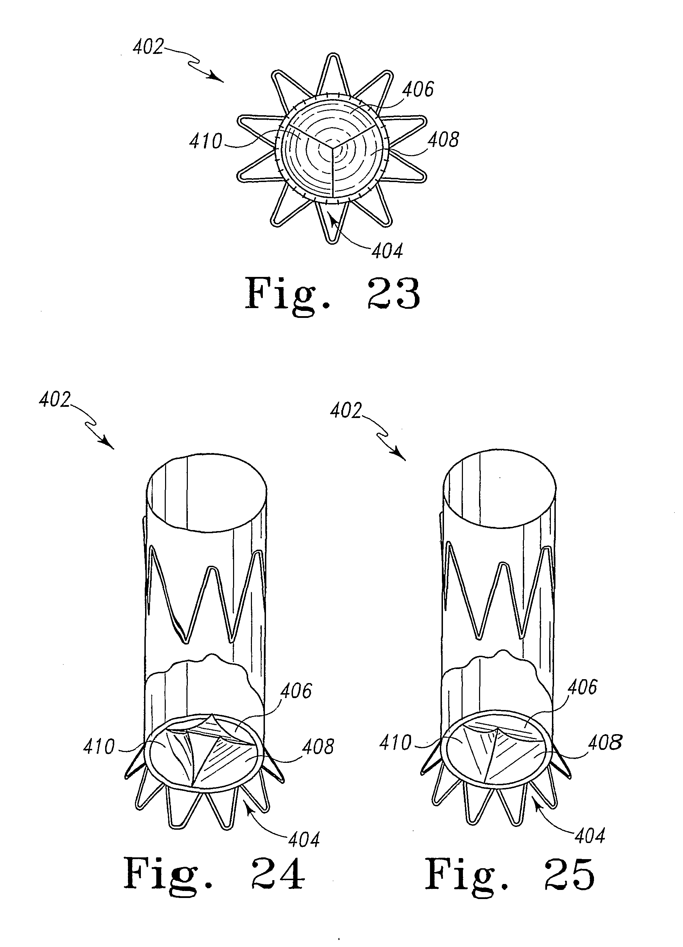

FIG 23 is a bottom (proximal) view of a third intraluminal prosthesis 402. The third intraluminal prosthesis 402 includes a valve prosthesis 404, but is otherwise similar to the intraluminal prosthesis 122. The valve prosthesis 404 can include a first leaflet 406, a second leaflet 408, and a third leaflet 410. The first leaflet 406, second leaflet 408, and third leaflet 410 can comprise any at least substantially biocompatible material including organic and inorganic materials described above.

FIG 24 is a cutaway perspective view of the intraluminal prosthesis 402 showing the valve prosthesis 404 located therein. The first, second, and third leaflets 406 - 408 can be arranged in the intraluminal prosthesis 402 such that they mimic a naturally occurring tricuspid valve. As shown in FIG 24, the valve prosthesis 404 is in an "open" position. As shown in FIG 25, however, the valve prosthesis 404 closes when fluid pressure on the distal side of the valve prosthesis 404 is greater than pressure on the proximal side of the valve prosthesis 404.

Throughout this specification, unless the context requires otherwise, the words "comprise" and "include" and variations such as "comprising" and "including" will be understood to imply the inclusion of an item or group of items, but not the exclusion of any other item or group items.

While various embodiments of the invention have been described, it will be apparent to those of ordinary skill in the art that many more embodiments and implementations are possible within the scope of the invention. Furthermore, although various indications have been given as to the scope of this invention, the invention is not limited to any one of these but can reside in two or more of these combined together. Accordingly, the invention is not to be restricted except in light of the attached claims and their equivalents.

## Claims

1. An endoluminal prosthesis for providing replacement for a damaged endoluminal valve for restricting fluid flow in a lumen, the prosthesis comprising:
a tubular graft (124) having a flexible body including an inner surface that defines an inner volume, a self-expanding stent (130) mounted to a proximal end of the flexible body, the self-expanding stent extending beyond the flexible body proximal end, and a valve prosthesis (138) at the proximal end of the flexible body, **characterised in that** the valve prosthesis including at least one leaflet (140, 142) having an edge (136) coupled to the proximal end of the flexible body, the or each leaflet having a free edge responsive to pressure differentials and movable between an open position permitting a fluid flow through the inner volume and a closed position restricting fluid flow through the inner volume.

2. The endoluminal prosthesis of claim 1 wherein the tubular graft (124) comprises a plurality of internal or external self-expanding stents (126) that are coupled along the length of the flexible body.

3. The endoluminal prosthesis of claim 1 wherein the tubular graft further comprises a second self-expanding stent (128) mounted to a distal end of the flexible body of the tubular graft (124) and extending beyond the said distal end.

4. The endoluminal prosthesis of claim 3 wherein the second self-expanding stent (128) includes attachment barbs.

5. The endoluminal prosthesis of claim 1 wherein the self-expanding stent (130) includes attachment barbs.

6. The endoluminal prosthesis of any preceding claim wherein the or each leaflet (140, 142) consists substantially of a biomaterial.

7. . The endoluminal prosthesis of claim 6 wherein the biomaterial comprises a derived collagen material.

8. The endoluminal prosthesis of claim 7 wherein the derived collagen material is an extracellular matrix.

9. The endoluminal prosthesis of claim 8 wherein the extracellular matrix is one of small intestinal submucosa; stomach submucosa; pericardium; liver basement membrane; urinary bladder submucosa; tissue mucosa or dura mater.

10. The endoluminal prosthesis of any preceding claim including sutures or rings coupling each leaflet (140, 142) to the tubulargraft (124).

11. The endoluminal prosthesis of any preceding claim wherein the tubular graft (124) is a body portion of a thoracic aortic prosthetic device, a renal prosthetic device a superior mesenteric prosthetic device or a celiac prosthetic device.

## Patentansprüche

1. Endoluminale Prothese zur Bereitstellung eines Ersatzes für eine geschädigte endoluminale Klappe zur Begrenzung der Flüssigkeitsströmung in einem Lumen, wobei die Prothese Folgendes umfasst:
ein röhrenförmiges Transplantat (124) mit einem flexiblen Körper mit einer Innenfläche, die ein Innenvolumen definiert, einem selbst-expandierenden Stent (130), der an einem proximalen Ende des flexiblen Körpers angebracht ist, wobei sich der selbst-expandierende Stent über das proximale Ende des flexiblen Körpers hinaus erstreckt, und einer Klappenprothese (138) an einem proximalen Ende des flexiblen Körpers, **dadurch gekennzeichnet, dass** die Klappenprothese zumindest ein Segel (140, 142) mit einem Rand (136), der mit dem proximalen Ende des flexiblen Körpers verbunden ist, aufweist, wobei das oder jedes Segel einen freien Rand aufweist, der auf Druckdifferenzen anspricht und zwischen einer geöffneten Stellung, in der eine Flüssigkeitsströmung durch das Innenvolumen gestattet ist, und einer geschlossenen Stellung, in der Flüssigkeitsströmung durch das Innenvolumen begrenzt wird, bewegbar ist.

2. Endoluminale Prothese nach Anspruch 1, worin das röhrenförmige Transplantat (124) eine Vielzahl von inneren oder äußeren selbst-expandierenden Stents (126) umfasst, die entlang der Länge des flexiblen Körpers verbunden sind.

3. Endoluminale Prothese nach Anspruch 1, worin das röhrenförmigen Transplantat ferner einen zweiten selbst-expandierenden Stent (128) umfasst, der an einem distalen Ende des flexiblen Körpers des röhrenförmigen Transplantats (124) angebracht ist und sich über das distale Ende hinaus erstreckt.

4. Endoluminale Prothese nach Anspruch 3, worin der zweite selbst-expandierende Stent (128) Befestigungswiderhaken aufweist.

5. Endoluminale Prothese nach Anspruch 1, worin der selbst-expandierende Stent (130) Befestigungswiderhaken aufweist.

6. Endoluminale Prothese nach einem der vorhergehenden Ansprüche, worin das oder jedes Segel (140, 142) im Wesentlichen aus einem biologischen Material besteht.

7. Endoluminale Prothese nach Anspruch 6, worin das biologische Material ein abgeleitetes Kollagenmaterial umfasst.

8. Endoluminale Prothese nach Anspruch 7, worin das abgeleitete Kollagenmaterial eine extrazelluläre Matrix ist.

9. Endoluminale Prothese nach Anspruch 8, worin die extrazelluläre Matrix Dünndarm-Submukosa; Magen-Submukosa; Perikard; Leber-Basalmembran; Harnblasen-Submukosa; Gewebeschleimhaut oder Dura mater ist.

10. Endoluminale Prothese nach einem der vorhergehenden Ansprüche, einschließlich Nähte oder Ringe, die jedes Segel (140, 142) mit dem röhrenförmigen Transplantat (124) verbinden.

11. Endoluminale Prothese nach einem der vorhergehenden Ansprüche, worin das röhrenförmige Transplantat (124) ein Körperabschnitt einer Prothesenvorrichtung für eine Aorta thoracica, einer Prothesenvorrichtung für eine Nierenarterie, einer Prothesenvorrichtung für eine Arteria mesenterica superior oder einer Prothesenvorrichtung für eine Arteria coeliaca ist.

## Revendications

1. Prothèse endoluminale destinée à fournir un remplacement d'une valve endoluminale endommagée pour limiter l'écoulement de fluide dans une lumière, la prothèse comprenant :
une greffe tubulaire (124) ayant un corps flexible comportant une surface interne qui définit un volume interne, un stent auto-expansible (130) monté sur une extrémité proximale du corps flexible, le stent auto-expansible s'étendant au-delà de l'extrémité proximale du corps flexible, et une prothèse de valve (138) à l'extrémité proximale du corps flexible, **caractérisée en ce que** la prothèse de valve comporte au moins un feuillet (140, 142) ayant un bord (136) accouplé à l'extrémité proximale du corps flexible, le ou chaque feuillet ayant un bord libre réagissant à des différences de pression et déplaçable entre une position ouverte permettant un écoulement de fluide à travers le volume interne et une position fermée limitant l'écoulement de fluide à travers le volume interne.

2. Prothèse endoluminale selon la revendication 1, dans laquelle la greffe tubulaire (124) comprend une pluralité de stents auto-expansibles internes ou externes (126) qui sont accouplés le long de la longueur du corps flexible.

3. Prothèse endoluminale selon la revendication 1, dans laquelle la greffe tubulaire comprend en outre un deuxième stent auto-expansible (128) monté sur une extrémité distale du corps flexible de la greffe tubulaire (124) et s'étendant au-delà de ladite extrémité distale.

4. Prothèse endoluminale selon la revendication 3, dans laquelle le deuxième stent auto-expansible (128) comporte des barbelures de fixation.

5. Prothèse endoluminale selon la revendication 1, dans laquelle le stent auto-expansible (130) comporte des barbelures de fixation.

6. Prothèse endoluminale selon l'une quelconque des revendications précédentes, dans laquelle le ou chaque feuillet (140, 142) est substantiellement constitué d'un biomatériau.

7. Prothèse endoluminale selon la revendication 6, dans laquelle le biomatériau comprend un matériau de collagène dérivé.

8. Prothèse endoluminale selon la revendication 7, dans laquelle le matériau de collagène dérivé est une matrice extracellulaire.

9. Prothèse endoluminale selon la revendication 8, dans lequel la matrice extracellulaire est l'une parmi une sous-muqueuse de l'intestin grêle ; une sous-muqueuse de l'estomac ; le péricarde ; la membrane basale du foie ; une sous-muqueuse de la vessie ; un tissu muqueux ou la dure-mère.

10. Prothèse endoluminale selon l'une quelconque des revendications précédentes, comportant des sutures ou des bagues accouplant chaque feuillet (140, 142) à la greffe tubulaire (124).

11. Prothèse endoluminale selon l'une quelconque des revendications précédentes, dans laquelle la greffe tubulaire (124) est une portion corporelle d'un dispositif prothétique thoracique aortique, d'un dispositif prothétique rénal, d'un dispositif prothétique supérieur mésentérique ou d'un dispositif prothétique coeliaque.
